# EUROPEAN PATENT SPECIFICATION

(11) **EP 4 517 101 B1**
(45) Date of publication and mention of the grant of the patent: **15.10.2025**
(21) Application number: 24197306.4
(22) Date of filing: 29.08.2024
(51) Int. Cl.: F04D 29/02, C02F 1/467, F04D 29/42, A61L 2/03

(54) **PREVENTION OF MICROBIOLOGICAL GROWTH IN PROCESSING EQUIPMENT HAVING A ROTATING HOUSING AND A ROTATING ELEMENT**
VERHINDERUNG VON MIKROBIOLOGISCHEM WACHSTUM IN VERARBEITUNGSAUSRÜSTUNG MIT EINEM ROTIERENDEN GEHÄUSE UND EINEM ROTIERENDEN ELEMENT
PRÉVENTION DE LA CROISSANCE MICROBIOLOGIQUE DANS UN ÉQUIPEMENT DE TRAITEMENT AYANT UN BOÎTIER ROTATIF ET UN ÉLÉMENT ROTATIF

(30) Priority: 29.08.2023 EP 23193833
(43) Date of publication of application: 05.03.2025
(73) Proprietor: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(56) References cited:
- WO-A1-2021/069395
- FR-A1- 2 697 443
- GB-A- 201 907
- US-A1- 2019 249 815

## Description

### Technical field

The technology proposed herein relates to the prevention of microbiological growth in food processing equipment.

### Background

Microbiological growth in food processing equipment is a recognized problem, for example in diary processing. The feed that is processed have a high nutrient content and the rate of microbiological growth in the equipment is typically high. Extensive biofilms can form in a few hours. Frequent cleaning is typically required to prevent a drop in quality of the produced product or in the performance of the equipment. For example, microbiological growth may contaminate the produced product, or it may obstruct the flow of the fluid within the equipment. Typically, production must be interrupted for cleaning and chemicals are commonly used, which contributes to a decreased efficiency and increased production costs.

Microbiological growth is particularly a problem in equipment that that cannot easily be disassembled for manual cleaning. Equipment having rotating components, such as decanter centrifuges are typically difficult to disassemble.

US2019/249815A1 discloses a system for preventing microbiological growth in a piping system.

### Object of the proposed technology

The proposed technology aims at preventing or reducing microbiological growth, such as the formation of biofilms, in food processing equipment. A particular object of the proposed technology is to reduce microbiological growth in equipment having a rotating housing and a rotating element.

### Summary

In a first aspect of the proposed technology, a system is proposed that comprises: a housing, or container, and an electric power source, or electric power supply. The housing is electrically conductive and arranged to, or configured to, hold, or contain, a fluid, the power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent, microbiological growth within the housing, or on the housing.

The housing is a rotating housing, or rotary housing. It is understood that the housing may be arranged to rotate around a housing axis, or first rotational axis. The system further comprises: a rotating element, or rotary element, wherein the rotating element is located at least partly within the housing. It is understood that the rotating element is arranged to rotate relative to the housing. It is further understood that the rotating element may rotate around a rotating-element axis, or second rotational axis. It is further understood that the rotating element and the housing may be electrically coupled in an electric network, and that the electric network may form part of the electric circuit. More generally, the rotating element may form part of the abovementioned electric circuit. It is understood that the electric circuit may be further arranged to lead the current from the electric power source through, or via, the rotating element, and/or to establish the potential over the rotating element. It is understood that the rotating element may be electrically conductive, and that the electric current, and/or the electric potential, may be further configured to inhibit, reduce, or prevent microbiological growth on the rotating element.

It is understood that the intention of the system is to prevent microbiological growth in the housing. It is further understood that the housing may form part of a food processing equipment.

It is further understood that the housing has a portion that is exposed to the fluid, and the electric circuit may be arranged to lead the current from the electric power source through, or via, the portion exposed to the fluid. It is further understood that the current is configured to inhibit microbiological growth on the housing or on the portion of the housing exposed to the fluid.

It is specified that the electric circuit is arranged to lead the current through the housing. It is understood that the housing extends in all dimensions and that the current may be evenly or unevenly distributed in the housing. It is further understood that at least a portion of the housing forms part of the electric circuit. This means that the current passes through, or via, this portion. The complete housing may form part of the circuit, which means that the current passes through the complete housing.

Throughout these specifications, if a first element is electrically coupled to a second element there may be further conductive elements between them, and if a first element is electrically connected to a second element there are no conductive elements between them.

The fluid may be susceptible to microbiological growth. The fluid may contain water. The fluid may be a liquid, such as raw milk or raw juice. It is understood that the fluid may contain solid particles. The fluid may be a paste, or a minced or pureed product.

The electric circuit may be connected to ground. Worded differently, the housing may be electrically coupled to the power source via ground, or the electric circuit may incorporate ground. Ground may be a signal ground or earth ground. For example, ground may incorporate an electrically conductive support structure connected to and supporting the housing.

The power source may have a first terminal and a second terminal. The first terminal may supply the current or the potential, and the second terminal may be, coupled, or connected, to ground, or vice versa. In this context, it is understood that the potential is relative to ground. The power source may be a single-phase power source. This means that it has a single terminal supplying the current or the potential, for example by the first terminal.

The current may be an alternating current. An alternating current is understood to periodically change direction. The current may have a square waveform. The current may be below 10 mA, below 1 mA, or below 0.1 mA. The current may be greater than 0.01 mA. The current may be in the range 10 mA to 1 mA, 1 mA to 0.1 mA, or 0.1 mA to 0.01 mA. Here, the current is understood as the absolute maximum over a period. It has been found that this current is sufficient for inhibiting microbiological growth. The alternating current may have a frequency below 100 Hz, below 10 Hz, or below 1 Hz. The frequency may be greater than 0.1 Hz. The alternating current may be supplied at a peak voltage below 120 V, in the range 10 V to 100 V, or in the range 20 V to 90 V, or in the range 30 V to 70 V.

Alternatively to the current being an alternating current, the current may be a direct current. A direct current is understood to have a constant direction. The direct current may be a pulsed current. It is understood that no current is supplied between pulses. The pulsed current may have a regular pulse frequency. The pulse frequency may be below 100 Hz, below 10 Hz, or below 1 Hz. The pulse frequency may be greater than 0.1 Hz. The direct current may have a duty cycle in the range 60% to 40%, or about 50%. For example, the current may be supplied with a pulse length of 500 ms with a pulse separation of 500 ms. The pulsed current may be supplied at a peak voltage below 120 V, in the range 10 V to 100 V, in the range 20 V to 90 V, or in the range 30 V to 70 V.

The housing may be of metal, such as steel. The housing may form, or enclose, an inner space for holding, or retaining, the fluid. The housing may have a wall forming the inner space for holding the fluid. It is understood that the wall is exposed to the fluid. The wall may be electrically conductive. For example, the wall may be of metal. It is understood that the housing, or wall, may be composed of several components that are connected, or joined, together. The wall may be, or form part of the abovementioned portion of the housing.

The housing may have, or form, an inlet, or feed nozzle, for receiving, or introducing, the fluid into the housing. The housing may have, or form, an outlet, or discharge nozzle, for discharging, or dispelling, the fluid from the housing. The inlet and the outlet may be located at opposite sides, or ends, of the housing. It is understood that the housing may be exposed to the fluid between the inlet and the outlet. The electric circuit may be connected to the housing at the inlet and at the outlet. This contributes to inhibit microbiological growth between the inlet and the outlet. If the inlet and the outlet are located at opposite sides of the housing, microbiological growth in the complete housing may be inhibited.

It is specified that the housing is a rotating housing, and that the housing may be arranged to rotate around a housing axis, or first rotational axis. The system may further comprise a support structure that rotationally supports the housing, for example by way of one or more support bearings. The housing may be electrically connected, or coupled, to the support structure, for example via the support bearings. Worded differently, the housing may be electrically coupled to the electric power source via the support structure, or more specifically via the support bearings, or the support structure, or more specifically the support bearings, may form part of the electric circuit. Alternatively, the housing may be electrically insulated from the support structure.

The housing may comprise a housing shaft. The housing shaft may be centred on the housing axis. The housing shaft may be a driven shaft, or transmission shaft. For example, the system may comprise a housing motor, wherein the housing motor is coupled to and arranged to rotate the housing shaft. It is understood that there may be parts of the housing, for example the housing shaft, that does not hold the fluid.

It is specified that the housing is electrically conductive. This means that the housing shaft is electrically conductive. For example, the housing shaft may be of metal, such as steel. It is specified that the electric circuit is arranged to lead the current through the housing. The electric circuit may be further arranged to lead the current through, or via, the housing shaft. Worded differently, the housing shaft may form part of the abovementioned electric circuit, or more specifically of the electric network.

It is specified that the system comprises a rotating element and that the rotating element is located at least partly within the housing. More specifically, the rotating element may be located at least partly in the inner space. It is understood that the rotating element may comprise several parts. Worded differently, the rotating element may be a composite structure. The parts may be fixed relative to one another. Worded differently, the rotating element may be a rigid structure.

It is specified above that the housing may be arranged to rotate around a housing axis. The housing axis and the rotating-element axis may be coaxial. Worded differently, the housing axis and the rotating-element axis may be the same rotational axis. It is understood that the housing and the rotating element may rotate at different rates. Additionally, they may rotate in the same direction or in different directions relative to the rotational axis.

The rotating element may extend from within the housing to outside the housing. The rotating element may in part be located within the abovementioned inner space formed by the housing. It may further be accessible from outside the housing. It is understood that the rotating element may be a rigid self-supporting structure. It is understood that the rotating element may conform to the housing, or more specifically to the wall formed by the housing.

The rotating element may form an inlet for introducing the fluid into the housing, or in the inner space formed by the housing. The inlet may be located within the housing, or in the inner space.

The rotating element may be, or comprise a rotating-element shaft, wherein the rotating-element shaft extends from within the housing to outside the housing. It is understood that the rotating-element shaft may be arranged to rotate around the rotating-element axis relative to the housing. The rotating-element shaft may be centred on the rotating-element axis. The rotating-element shaft may be a driven shaft, or transmission shaft. For example, the system may comprise a rotating-element motor, wherein the rotating-element motor is coupled to and arranged to rotate the rotating-element shaft. It is understood that the rotating-element motor may be connected directly to the rotating-element shaft, or that it may be connected to the rotating-element shaft via additional components, for example via additional shafts and joints. For example, the rotating-element shaft may be fixed to the rest of the rotating element.

The rotating element may be electrically conductive. For example, the rotating element may be of metal, such as steel. It is specified that the rotating element may form part of the electric circuit. More specifically, the rotating-element shaft may form part of the abovementioned electric circuit. The rotating-element shaft may be exposed to the fluid. This way, the electric current, and/or the electric potential, is further configured to inhibit, reduce, or prevent microbiological growth on the rotating-element shaft.

The electric circuit may be arranged to lead the current through, or via, both the housing and the rotating element, and/or to establish the potential over the housing and the rotating element. Worded differently, the electric circuit may be arranged to lead the current through, or via, the combined housing and rotating element, and/or to establish the potential over the combined housing and rotating element. The electric circuit may be arranged to lead the current through and/or to establish the potential over at least a portion of the housing and/or over at least a portion of the rotating element. This means that there may be parts of the housing and/or the rotating element through which the current does not run. The electric circuit may be arranged to lead the current through and/or to establish the potential over the complete housing and/or the complete rotating element.

As specified above, the rotating element and the housing may be electrically coupled in an electric network. The electric network may be arranged to distribute the current between the housing and the rotating element. For example, the housing and the rotating element may be arranged in parallel in the electric network. This means that in the electric network, a first portion of the current is conducted through, or via, the housing and a second portion of the current is conducted through, or via, the rotating element.

The rotating element, or the rotating-element shaft, may be electrically coupled to the housing. The rotating element, or the rotating-element shaft, may contact the housing. It is understood that this may establish a direct electric contact between the rotating element, or rotating-element shaft, and the housing.

The system may comprise a first internal connector, or first internal connector arrangement, that electrically couples the rotating element, or the rotating-element shaft, to the housing. The system may further comprise a second internal connector, or second internal connector arrangement, that electrically couples the rotating element, or the rotating-element shaft, to the housing. Any of the first internal connector and the second internal connector may be a rotary electrical connector, or electrical rotary joint. Here, rotary electrical connectors are understood to allow a transmission of electrical power or electrical signals from a stationary structure to a rotating structure.

The first internal connector and the second internal connector may be spaced apart along the rotating-element axis. They may be centred on the rotating-element axis. This contributed to a more even distribution of the current, and in extension a to more even reduction of microbiological growth.

For example, any of the internal connectors may comprise a slip ring connecting the rotating element, or the rotating-element shaft, and the housing. Additionally or alternatively, any of the internal connectors may comprise a bearing, such as a metal rolling-element bearing or a metal plain bearing connecting the rotating element, or the rotating-element shaft, and the housing. Additionally or alternatively, any of the internal connectors may comprise a metal brush or carbon brush connecting the rotating element, or the rotating-element shaft, and the housing. Additionally or alternatively, any of the internal connectors may comprise a wear ring attached to the rotating element or the housing connecting the rotating element and the housing. Additionally or alternatively, any of the internal connectors may comprise an electrically conductive seal, for example of metal, connecting the rotating element, or the rotating-element shaft, and the housing. It is understood that the seal may be arranged to, or configured to, inhibit, or prevent, a fluid flow between the rotating element and the housing. A fluid is here understood to included liquids and gases. It is further understood that such a seal is a mechanical seal, or a device that provides a sealing at a point of entry or exit of a rotating element.

The rotating element, or the rotating-element shaft, may be connected to the electric circuit via the housing. For example, the abovementioned second portion of the current may pass through, or via, the housing before and/or after passing through, or via, the rotating element. The housing may be connected to the electric circuit via the rotating element, or via the rotating-element shaft. For example, the abovementioned first portion of the current may pass through, or via, the rotating element before and/or after passing through, or via, the housing.

The system may further comprise: a static element, or a stationary element. The static element may be located at least partly within the housing, or in the inner space. Alternatively, the complete static element may be located outside the housing, or outside the inner space. It is understood that the housing may be arranged to rotate relative to the static element. It is understood that the static element may be a rigid self-supporting structure.

It is specified above that the housing is a rotating housing arranged to rotate around a housing axis. The housing axis may extend through the static element. The static element may be centred on the housing axis. Worded differently, the static element and the housing shaft may be concentric.

The static element may extend from within the housing, or the inner space, to outside the housing. The static element may extend through, or via, the housing shaft. The static element may in part be located in the abovementioned inner space formed by the housing. It may further be accessible from outside the housing.

The static element may form an inlet for introducing the fluid into the housing, or in the inner space formed by the housing. The inlet may be located within the housing or in the inner space. It is understood that the inner space may be in fluid communication with the inlet.

The static element may be electrically conductive. For example, the static element may be of metal, such as steel. The static element may form part of the abovementioned electric circuit. This way, the electric current, and/or the electric potential, may be further configured to inhibit, reduce, or prevent microbiological growth on the static element. The electric circuit may be arranged to lead the current through, or via, both the housing and the static element, and/or to establish the potential over the housing and the static element. Worded differently, the electric circuit may be arranged to lead the current through, or via, the combined housing and static element, and/or to establish the potential over the combined housing and static element. The electric circuit may be arranged to lead the current through and/or to establish the potential over at least a portion of the housing and/or over at least a portion of the static element. This means that there may be parts of the housing and/or the static element through which the current does not run. The electric circuit may be arranged to lead the current through and/or to establish the potential over the complete housing and/or the complete static element.

It is specified that the rotating element and the housing may be electrically coupled in an electric network. The static element may be electrically coupled in the electric network. The electric network may be arranged to distribute the current between the housing, the rotating element, and the static element. For example, the housing and the rotating element may be arranged in parallel in the electric network, the housing and the static element may be arranged in series in the electric network, and the rotating element and the static element may be arranged in series in the electric network. This means that in the electric network, a first portion of the current is conducted through, or via, the housing, a second portion of the current is conducted through, or via, the rotating element, and the total current is conducted through, or via, the static element.

The system may comprise a third internal connector, or third internal connector arrangement, that electrically couples the static element to the rotating element. The third internal connector may be located between the first internal connector and the second internal connector along the rotating-element axis.

For example, the third internal connector may comprise a slip ring connecting the static element and the rotating element. Additionally or alternatively, the third internal connector may comprise a bearing, such as a metal rolling-element bearing or a metal plain bearing connecting the static element and the rotating element. Additionally or alternatively, the third internal connector may comprise a metal brush or carbon brush connecting the static element and the rotating element. Additionally or alternatively, the third internal connectors may comprise a wear ring attached to the static element or the rotating element and connecting the static element and the rotating element. Additionally or alternatively, the third internal connector may comprise an electrically conductive seal, for example of metal, connecting the static element and the rotating element. It is understood that the seal may be arranged to, or configured to, inhibit, or prevent, a fluid flow between the static element and the rotating element.

The static element may be connected to the electric circuit via the housing and/or the rotating element, or vice versa. For example, the abovementioned first portion or second portion of the current may pass through, or via, the housing or rotating element before and/or after passing through, or via, the static element, or vice versa.

The system may further comprise: a first connector, or first connector arrangement, wherein the housing is electrically coupled to the power source via the first connector. The first connector may be connected to the housing, the rotating element, or the static element. This means that the first connector may form part of the abovementioned electric circuit. If the first connector is connected to the housing or the rotating element, the first connector may be a rotary electrical connector. If the first connector is connected to the static element, the first connector may be a static connector. The system may further comprise: a second connector, or second connector arrangement, wherein the housing is electrically coupled to the power source via the second connector. The second connector may be connected to the housing, the rotating element, or the static element. This means that the second connector may form part of the abovementioned electric circuit. If the second connector is connected to the housing or the rotating element, the second connector may be a rotary electrical connector. If the second connector is connected to the static element, the second connector may be a static connector. The abovementioned support structure may constitute, or form part of, the first connector or the second connector, and the support structure may be connected to ground.

The first connector and the second connector may be positioned on opposite sides of the housing, or the inner space. The abovementioned inlet and/or outlet may be located between the first connector and the second connector. This contributes to inhibit microbiological growth within a greater part of the housing.

The abovementioned first internal connector and/or second internal connector may be located between the first connector and the second connector, for example along the housing axis and the rotating-element axis. This contributes to a inhibit microbiological growth over a greater part of the rotating element, static element, or housing.

The system may further comprise an electric first wire, and the first connector may be connected to the power source by the first wire. The first wire may be connected to the abovementioned first terminal of the power source. Alternatively, the first connector and the power source may be connected via ground, for example via the abovementioned support structure. The system may further comprise an electric second wire, and the second connector may be connected to the power source by the second wire. The second wire may be connected to the abovementioned second terminal of the power source. Alternatively, the second connector and the power source may be connected via ground, for example via the abovementioned support structure. It is understood that the first connector and the second connector may not both be connected to the power source via ground.

It is specified that the housing is a rotating housing. The first connector may be connected to the housing, or to the housing shaft. Similarly, the second connector may be connected to the housing, or to the housing shaft. With this configuration, any of the first connector and the second connector may be a rotary electrical connector, or an electrical rotary joint.

It is further specified that the system comprises a rotating element and/or a static element. If both the first connector and the second connector are connected to the housing, the current may then be distributed to the rotating element and/or static element via the abovementioned first internal connector and second internal connector. This way, the electric current, and/or the electric potential, may be further configured to inhibit, reduce, or prevent microbiological growth on the rotating element and/or static element. It is understood that the electric circuit may be arranged to lead the current through, or via, the combined housing, rotating element and static element, and/or to establish the potential over the combined housing, rotating element, and static element.

The first connector and the second connector may be spaced apart along the housing axis. This contributes to a inhibit microbiological growth over a greater part of the rotating element or static element, particularly if the first internal connector and the second internal connector are spaced apart along the housing axis.

It is specified that the housing is a rotating housing. For example, the rotary electrical connector may comprise a slip ring connecting to the housing, or to the housing shaft. Additionally or alternatively, it may comprise a bearing, such as a metal rolling-element bearing or a metal plain bearing connecting to the housing, or to the housing shaft. For example, the bearing may have an inner race connected to the housing, or to the housing shaft and an outer race connected to the abovementioned support structure. Additionally or alternatively, the rotary electrical connector may comprise a metal brush or carbon brush connecting to the housing, or to the housing shaft. Additionally or alternatively, it may comprise a wear ring connecting to the housing, or to the housing shaft. Additionally or alternatively, the rotary electrical connector may comprise an electrically conductive seal, for example of metal, connecting to the housing, or to the housing shaft.

It is specified that the system comprises a rotating element. The first connector may be connected to the rotating element, or to the rotating-element shaft. Similarly, the second connector may be connected to the rotating element, or to the rotating-element shaft. Any of the first connector and the second connector may connect to the rotating element outside the housing, for example to the rotating-element shaft. With this configuration, any of the first connector and the second connector may be a rotary electrical connector, or an electrical rotary joint. If both the first connector and the second connector are connected to the rotating element, the current may then be distributed to the housing via the abovementioned first internal connector and second internal connector. The first connector and the second connector may be spaced apart along the rotating-element axis. This contributes to a inhibit microbiological growth over a greater part of the rotating element.

For example, the rotary electrical connector may comprise a slip ring connecting to the rotating element, or to the rotating-element shaft. Additionally or alternatively, it may comprise a bearing, such as a metal rolling-element bearing or a metal plain bearing connecting to the rotating element, or to the rotating-element shaft. For example, the bearing may have an inner race connected to the rotating element, or to the rotating-element shaft, and an outer race connected to the abovementioned support structure. Additionally or alternatively, the rotary electrical connector may comprise a metal brush or carbon brush connecting to the rotating element, or to the rotating-element shaft. Additionally or alternatively, it may comprise a wear ring connecting to the rotating element, or to the rotating-element shaft. Additionally or alternatively, it may comprise an electrically conductive seal, for example of metal, connecting to the rotating element, or to the rotating-element shaft.

It is specified that the housing is a rotating housing and that the system additionally may comprise a static element. The first connector and/or the second connector may be connected to the static element. In this configuration, any of the first connector and the second connector may be a static connector, or a fixed connector. This means that the connector cannot move relative to the static element. For example, the static connector may be a ring lug connector secured to the static element by a screw, or it may be a clamp secured to the static element. If both the first connector and the second connector are connected to the static element, the current may then be distributed to the housing via the abovementioned first internal connector and second internal connector.

The housing and the rotating element may form part of a decanter, or decanter centrifuge. Wording the first aspect of the proposed technology differently, the system comprises: a decanter, or decanter centrifuge, that has, or comprises, a housing and a rotating element, and an electric power source, or electric power generator. The housing is rotating housing, the housing is electrically conductive and arranged to, or configured to, hold, or contain, a fluid, the power source is arranged to supply an electric current, and/or an electric potential, and the housing and the electric power source are operationally coupled in an electric circuit arranged to lead the current from the electric power source through, or via, the housing, and/or to establish the potential over the housing, and the electric current, and/or the electric potential, is configured to inhibit, reduce, or prevent microbiological growth within the housing, or on the housing. It is understood that the rotating element is arranged to rotate relative to the housing.

It is understood that the system may have any of the features described above. For example, the housing may have a housing shaft, and the rotating element may have a rotating-element shaft. The housing axis and the rotating-element axis may be the same rotational axis. Worded differently, the housing shaft and the rotating-element shaft may be coaxial. The housing axis, and in extension the rotational axis, may be horizontal. Further, the rotating element and the housing may be electrically coupled in an electric network, and the electric network may form part of the electric circuit.

The first internal connector, second internal connector, and/or third internal connector may form part of the decanter. Worded differently, the decanter may comprise the first internal connector, second internal connector, and/or third internal connector.

As described above, the system may comprise: a static element. The static element may form part of the decanter centrifuge. Worded differently, the decanter centrifuge may have, or comprise, a static element. The static element may have any of the features described above.

The static element may form an inlet for introducing the fluid into the housing. The inlet may be located within the housing, or in the inner space. The static element may extend into the rotating element. The inlet may be located inside the rotating element. Worded differently, the static element, or the inlet, may be configured to release the fluid inside the rotating element. The rotating element may form a passage configured to release the fluid into the housing, or the inner space. Worded differently, the passage may fluidly connect the static element and the inner space, or the passage may be configured to release the fluid outside the rotating element (68). Alternatively, the rotating element may form an inlet, and the inlet may be located within the housing or in the inner space.

It is understood that the housing may form an opening, and that the static element may extend through the opening. More specifically, the housing shaft may be hollow, or form a through hole. The static element may extend through the housing shaft, for example through the through hole.

The static element may comprise, or form, an inlet pipe extending into the housing or into the inner space, for example via the housing shaft. It is understood that the inlet pipe is accessible from outside the housing. The inlet pipe may extend into the rotating element. The static element, or the inlet pipe, may have an outer open end for receiving the fluid and an inner open end constituting, or forming, the inlet. It is understood that the outer open end is accessible from outside the housing. The inner open end may be located within the housing, or more specifically within the rotating element.

It is specified above that the housing may have an outlet. It may further have an additional outlet, or additional discharge nozzle, for discharging, or dispelling, the fluid from the housing. The decanter, or more generally the system, may be arranged to, or configured to, form a lighter phase, or low-density fraction, of the fluid and a heavier phase, or a high-density fraction, of the fluid within the housing, or in the inner space, at a rotation of the housing. The outlet may be arranged to, or configured to, discharge the lighter phase and the additional outlet may be arranged to, or configured to, discharge the heavier phase.

The housing may have a first end and an opposite second end, wherein the first end and the second end are located at the housing axis. The outlet may be located at the first end and the additional outlet may be located at the second end. The inner space of the housing, or the wall of the housing, may outline a cylinder at the first end, and it may taper towards the housing axis at the second end. It is specified above that the rotating element may comprise a rotating-element shaft. The rotating-element shaft may be located at the first end and the housing shaft may be located at the second end. The static element may be located at the second end. It specified above that each of the housing shaft and the rotating-element shaft may be driven shafts. It is further specified that the housing and the rotating element may rotate at different rates, and that they may rotate in the same directions.

The rotating element may further comprise a rotor, scroll, or conveyer, wherein the rotor is attached to, or fixed to, the rotating-element shaft and arranged to, or configured to, generate a flow of the heavier phase of the fluid, or to push the heavier phase of the fluid, to the additional outlet, towards the second end of the housing, or along the rotating-element axis, at a rotation around the rotating-element axis. The rotor may contact the housing, or the wall of the housing. Worded differently, the rotor may be slidably connected to, or electrically connected to, the housing, or the wall of the housing. Alternatively, the rotor may be spaced apart from, or have a clearance to, the housing, or the wall of the housing. It is understood that this is in operation of the system, or more specifically of the decanter.

In a specific application, the first internal connector connects, or couples, the rotating-element shaft to the housing and the rotor contacts the housing. For example, the first internal connector may comprise a bearing, such as a metal rolling-element bearing or a metal plain bearing, that connects the rotating-element shaft and the housing. The first connector connects to the housing and the second connector connects to the housing. The first internal connector and the rotor may be located between the first connector and the second connector. It is understood that his is along the housing axis or the rotating-element axis. The first internal connector may be located at first end of the housing. The first connector may be located at the first end of the housing and the second connector may be located at the second end. For example, each of the first connector and second connector may comprise a slip ring. The rotor may be located between the first connector and the second connector.

In another specific application, the abovementioned second internal connector further connects the rotor to the housing. For example, the second internal connector may be a bearing, such as a metal rolling-element bearing or a metal plain bearing, that connects the rotor and the housing, or more specifically the housing shaft. The second internal connector may be located at the second end of the housing. The rotor may be located between the first internal connector and the second internal connector.

In another specific application, the first internal connector and the second internal connector connect, or couple, the rotating element to the housing and the rotor has a clearance to the housing. For example, each of the first internal connector and the second internal connector may comprise a bearing, such as a metal rolling-element bearing or a metal plain bearing, that connects the rotating element and the housing. The system may further comprise a third internal connector, or third internal connector arrangement, that electrically couples the static element to the rotating element. For example, the third internal connector may comprise an electrically conductive seal or plain bearing. The first connector connects to the rotating element and the second connector connects to the static element. For example, the first connector may comprise a slip ring and the second connector may comprise a clamp secured to the static element. The first internal connector, second internal connector, and third internal connector may be located between the first connector and the second connector. The first connector may be located at the first end of the housing and the second connector may be located at the second end of the housing. The rotor may be located between the first internal connector and the second internal connector. The first internal connector may be located at first end of the housing and the second internal connector may be located at the second end of the housing.

In another specific application, the first internal connector and the second internal connector connect, or couple, the rotating element to the housing and the rotor has a clearance to the housing. For example, each of the first internal connector and the second internal connector may comprise a bearing, such as a metal rolling-element bearing or a metal plain bearing, that connects the rotating element and the housing. The system may further comprise a third internal connector, or third internal connector arrangement, that electrically couples the static element to the rotating element. For example, the third internal connector may comprise an electrically conductive seal or plain bearing. The first connector connects to the housing and the second connector connects to the static element. For example, the first connector may comprise a bearing, such as a metal rolling-element bearing or a metal plain bearing, that connects the housing and the support structure, and the first connector is coupled to the electric power source via ground. The second connector may comprise a clamp secured to the static element. The first internal connector, second internal connector, and third internal connector may be located between the first connector and the second connector. The first connector may be located at the first end of the housing and the second connector may be located at the second end of the housing. The rotor may be located between the first internal connector and the second internal connector. The first internal connector may be located at first end of the housing and the second internal connector may be located at the second end of the housing.

In a second aspect of the proposed technology, which is not a part of the claimed invention, a method of inhibiting, reducing, or preventing microbiological growth in the system, or more specifically in the housing of the system, according to the first aspect is proposed. The method comprises: operating the power source to supply the current.

### Brief description of the drawings

A more complete understanding of the abovementioned and other features and advantages of the technology proposed herein will be apparent from the following detailed description of preferred embodiments in conjunction with the appended drawings, wherein:
Fig. 1 is a schematic view of a system with a decanter centrifuge,
Fig. 2 is a schematic view of a system with another decanter centrifuge, and
Fig. 3 is a schematic view of a system with another decanter centrifuge.

### Detailed description

In the figures and the below description, the same reference numerals are used for the same or related features.

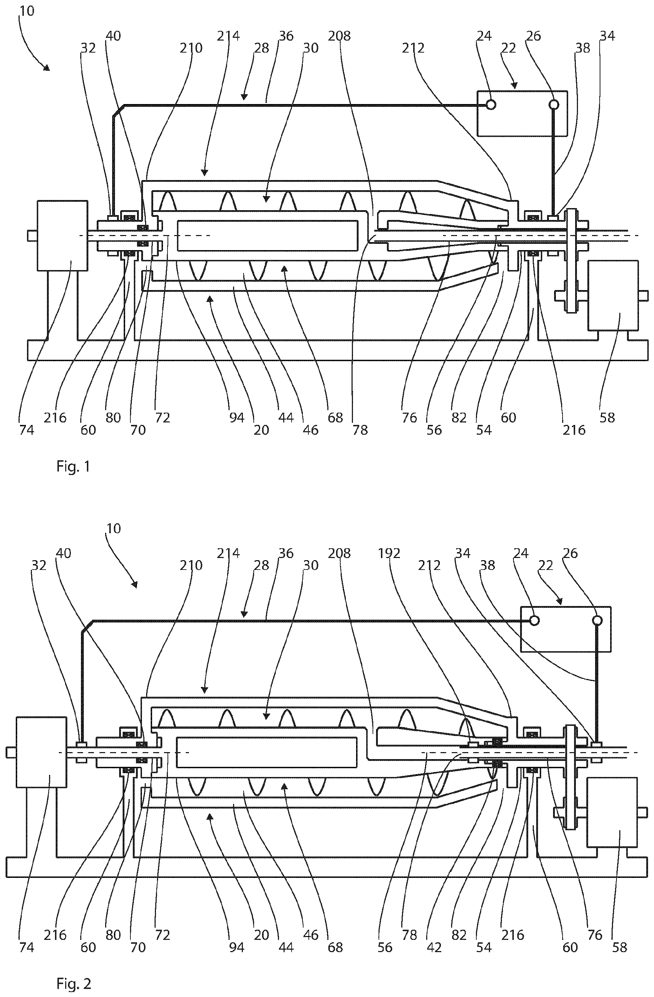

**Fig. 1** shows an embodiment of a system 10 including a decanter centrifuge 214 and an electric power source 22. The decanter centrifuge 214 has a housing 20 of steel that is electrically conductive and configured to hold a fluid. The housing 20 has an electrically conductive wall 44 exposed to the fluid and that forms an inner space 46 holding the fluid. The housing 20 is rotating housing that can rotate around a horizontal housing axis 56. The housing 20 is essentially rotationally symmetric relative to the housing axis 56. The housing 20 has a housing shaft 54 that is centred on the housing axis 56. The system 10 further has a support structure 60 that rotationally supports the housing 20. The housing 20 rotationally connected to the support structure 60 by support bearings 216 in the form of rotating element bearings. The system 10 further has a housing motor 58 coupled to the housing shaft 54 by which the housing 20 can be rotated. This means that the housing shaft 54 is a driven shaft.

The system 10 further has a rigid rotating element 68 composed of a rotating-element shaft 70 and a rotor 94 that are centred on and can rotate around a rotating-element axis 72 that is coaxial with the housing axis 56. The rotating element 68 is of steel and is electrically conductive. The rotating element 70 is in part located in the inner space 46 and the rotating-element shaft 70 extends from within the housing 20 to outside the housing 20, which means that it is accessible from outside the housing 20. The system 10 has a rotating-element motor 74 that is connected to can rotate the rotating-element shaft 70. This means that the rotating-element shaft 70 is a driven shaft.

The system 10 further has a static element 76 in the form of an inlet pipe that is centred on the housing axis 56. The static element 76 is stationary in operation of the centrifuge separator 16. The housing shaft 54 is hollow, the static element 76 extends through the housing shaft 54 into the rotating element 68. The static element 76 forms an inlet 78 that is located within the housing 20 and can release the fluid inside the rotating element 68. The rotating element 68 forms a passage 208 for the fluid from the inlet 78 to outside the rotating element 68. This way, the fluid can be introduced in the inner space 46. The static element 76 is of steel and is electrically conductive.

The housing 20 has a first end 210 and an opposite second end 212 at the rotating-element axis 72 respective the housing axis 56. The housing 20 forms an outlet 80 at the first end 210 and an additional outlet 82 at the second end 212 that can dispel the fluid from the housing 20. The decanter centrifuge 214 is configured to form a lighter phase and a heavier phase of the fluid within the housing 20 at a rotation of the housing 20 around the housing axis 56.

The inner space 46 of the housing 20 outlines a cylinder at the first end 210, and it tapers towards the housing axis 56 at the second end 212. The rotor 94 of the rotating element 68 conforms to the wall 44 of the housing 20. The rotating-element shaft 70 is located at the first end 210 and the housing shaft 54 is located at the second end 212. The housing 20 and the rotating element 68 rotate at different rates and in the same direction. The rotor 94 forms a scroll that generates a flow of the heavier phase of the fluid towards the second end 212 and the additional outlet 82 at a rotation around the rotating-element axis 72. The lighter phase of the fluid is discharged via the outlet 80, which is located radially inside of the of the additional outlet 82 relative to the housing axis 56. This way, the outlet 80 is configured to discharge the lighter phase, and the additional outlet 82 is configured to discharge the heavier phase.

The system 10 has a first connector 32 and a second connector 34 that are rotary electrical connectors in the form of electrically conductive slip rings. The first connector 32 and the second connector 34 are connected to housing 20 at the first end 210 respective the second end 212, which means that they are positioned on opposite sides of the housing 20 and the inner space 46. It further means that the inlet 78 and outlet 80 are located between the first connector 32 and the second connector 34.

The system 10 has a first internal connector 40 that is located at the first end 210 of the housing 20. The first internal connector 40 is a rotary electrical connector in the form of a metal rolling-element bearing that rotationally connects and electrically couples the rotating-element shaft 70 of the rotating element 68 and the housing 20. The rotor 94 of the rotating element 68 contacts the wall 44 of the housing 20 when operating the decanter centrifuge 214, and the rotor 94 is electrically connected to the housing 20. This way, the rotating element 68 and the housing 20 are electrically coupled in an electric network 30 between the first connector 32 and the second connector 34 via the first internal connector 40 and the contacting rotor 94.

The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal 26 is connected to ground and the second connector 34 is connected to ground via the support structure 60.

With the power source 22, first wire 36, first connector 32, first internal connector 40, rotor 94, housing 20, second connector 34, and second wire 38 arranged as described above, the housing 20, the rotating element 68, and the electric power source 22 are operationally coupled in an electric circuit 28 that can establish an electrical potential over the housing 20 and the rotating element 68 and that can lead an electric current from the electric power source 22 and through the housing 20 and the rotating element 68. The abovementioned electric network 30 forms part of the electric circuit 28.

The power source 22 can supply an alternating current with a square waveform. The current is about 0.5 mA, has a frequency below of 1 Hz, and is supplied at a peak voltage of 50 V. Each of the housing 20 and the rotating element 68 has a portion that is exposed to the liquid and forms part of the electric circuit 28. Thus, the electric circuit 28 can lead the current from the power source 22 through the portions exposed to the liquid, which inhibits microbiological growth on these components. This way, the supplied current contributes to reduce microbiological growth in the decanter centrifuge 214, and more precisely on the housing 20 and the rotating element 68.

**Fig. 2** shows another embodiment of a system 10 including a decanter centrifuge 214 and an electric power source 22. The system 10 differs from the system described in relation to Fig. 1 in that the first connector 32 is connected to the rotating-element shaft 70, the rotor 94 has a clearance to the housing 20, and the second connector 34 is a clamp secured to the static element 76 outside the housing 20. The system 10 further has a second internal connector 42 that is a rotary electrical connector in the form of a metal rolling-element bearing that rotationally connects and electrically couples the rotating element 68 and the housing 20. It further has a third internal connector 192 that is a rotary electrical connector in the form of a combined metal wear ring and seal that rotationally connects and electrically couples the rotating element 68 and the static element 76.

The second internal connector 42 is located at the second end 212 of the housing 20. The first internal connector 40, second internal connector 42, and third internal connector 192 are positioned between the first connector 32 and the second connector 34 in a direction along the housing axis 56. This way, the rotating element 68 and the housing 20 are coupled in parallel in an electric network 30 between the first connector 32 and the second connector 34 that distributes the current between the housing 20 and the rotating element 68 in a first portion that is conducted via the housing 20 and a second portion that is conducted via the rotating element 68. The static element 76 is coupled in series relative to the rotating element 68 and the housing 20.

With the power source 22, first wire 36, first connector 32, first internal connector 40, rotor 94, housing 20, second internal connector 42, static element 76, third internal connector 192, second connector 34, and second wire 38 arranged as described above, the housing 20, the rotating element 68, the static element 76, and the electric power source 22 are operationally coupled in an electric circuit 28 that can establish an electrical potential over the housing 20 and the rotating element 68 and lead the electric current from the electric power source 22 and through the housing 20 and the rotating element 68. This way, the supplied current contributes to reduce microbiological growth in the decanter centrifuge 214, and more precisely on the housing 20, rotating element 68, and static element 76.

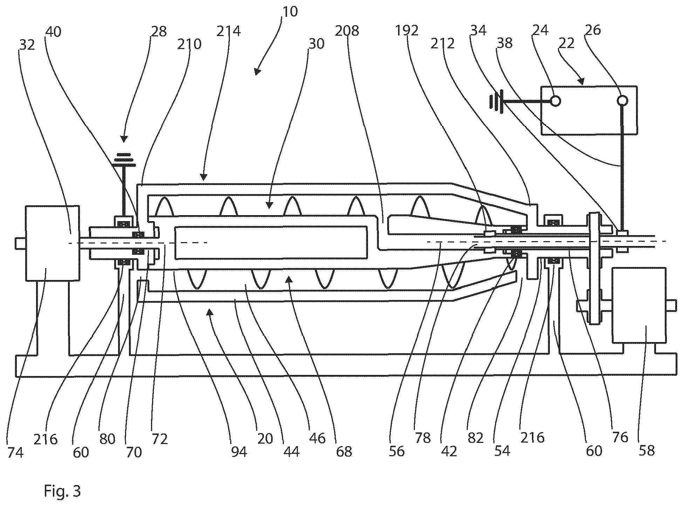

**Fig. 3** shows another embodiment of a system 10 including a decanter centrifuge 214 and an electric power source 22. The system 10 differs from the system described in relation to Fig. 2 in that the first connector 32 is a support bearing that electrically couples the housing 20 to the support structure 60. The support structure 60 and the first terminal 24 of the power source 22 are connected via ground.

With the power source 22, support structure 60, first connector 32, first internal connector 40, rotor 94, housing 20, second internal connector 42, static element 76, third internal connector 192, second connector 34, and second wire 38 arranged as described, the housing 20, the rotating element 68, the static element, and the electric power source 22 are operationally coupled in an electric circuit 28 that can establish an electrical potential over the housing 20, the rotating element 68, and static element 76, and lead the electric current from the electric power source 22 and through the housing 20, the rotating element 68, and the static element 76. This way, the supplied current contributes to reduce microbiological growth in the decanter centrifuge 214, and more precisely on the housing 20, rotating element 68, and static element 76.

### Item list

- 10: system
- 20: housing
- 22: electric power source
- 24: first terminal
- 26: second terminal
- 28: electric circuit
- 30: electric network
- 32: first connector
- 34: second connector
- 36: electric first wire
- 38: electric second wire
- 40: first internal connector
- 42: second internal connector
- 44: wall
- 46: inner space
- 54: housing shaft
- 56: housing axis
- 58: housing motor
- 60: support structure
- 68: rotating element
- 70: rotating-element shaft
- 72: rotating-element axis
- 74: rotating-element motor
- 76: static element
- 78: inlet
- 80: outlet
- 82: additional outlet
- 94: rotor
- 192: third internal connector
- 208: passage
- 210: first end
- 212: second end
- 214: decanter centrifuge

## Claims

1. A system (10) comprising:
- a housing (20), and
- an electric power source (22), wherein
the housing (20) is electrically conductive and configured to hold a fluid, the power source (22) is arranged to supply an electric current, the housing (20) and the electric power source (22) are operationally coupled in an electric circuit (28) arranged to lead the current from the electric power source (22) through the housing (20), and the electric current is configured to inhibit microbiological growth within the housing (20),
**characterized in that** the housing (20) is a rotating housing (20) arranged to rotate around a housing axis (56), and the system (10) further comprises:
- a rotating element (68), wherein
the rotating element (68) is located at least partly within the housing (20), the rotating element (68) is arranged to rotate around a rotating-element axis (72), and the rotating element (68) and the housing (20) are electrically coupled in an electric network (30) that forms part of the electric circuit (28).

2. The system (10) according to claim 1, wherein the system (10) further comprises:
- a first internal connector (40) that electrically couples the rotating element (68) to the housing (20), and the first internal connector (40) is a rotary electrical connector.

3. The system (10) according to claim 1 or 2, wherein the rotating element (68) comprises:
- a rotating-element shaft (70), and
- a rotor (94), wherein
the rotating-element shaft (70) extends from within the housing (20) to outside the housing (20) and is arranged to rotate around the rotating-element axis (72) relative to the housing (20), and the rotor (94) is attached to the rotating-element shaft (70) and configured to generate a flow of the fluid along the rotating-element axis (72) at a rotation around the rotating-element axis (72).

4. The system (10) according claim 3, wherein the system (10) further comprises:
- a second internal connector (42) that electrically couples the rotating element (68) to the housing (20), the second internal connector (42) is a rotary electrical connector, and the rotor (94) is located between the first internal connector (40) and the second internal connector (42).

5. The system (10) according to any of the claims 1 to 4, wherein the system (10) further comprises:
- a static element (76), wherein
the static element (76) and the housing (20) are electrically coupled in the electric network (30).

6. The system (10) according to claim 5, wherein the system (10) further comprises:
- a third internal connector (192) that electrically couples the static element (76) to the rotating element (68).

7. The system (10) according to any of the claims 1 to 6, wherein the electric circuit (28) is connected to ground.

8. The system (10) according to any of the claims 1 to 7, wherein the current is an alternating current, the current has a square waveform, the current is below 10 mA, and the current is greater than 0.01 mA.

9. The system (10) according to any of the claims 1 to 8, wherein the housing (20) and the rotating element (68) form part of a decanter (214), and the decanter (214) is arranged to form a lighter phase of the fluid and a heavier phase of the fluid within the housing (20) at a rotation of the housing (20).

## Patentansprüche

1. System (10), umfassend:
- ein Gehäuse (20) und
- eine elektrische Stromquelle (22), wobei
das Gehäuse (20) elektrisch leitfähig und zum Halten eines Fluids konfiguriert ist, die Stromquelle (22) zum Liefern eines elektrischen Stroms angeordnet ist, das Gehäuse (20) und die elektrische Stromquelle (22) in einem elektrischen Stromkreis (28) wirkgekoppelt sind, der so angeordnet ist, dass er den Strom von der elektrischen Stromquelle (22) durch das Gehäuse (20) leitet, und der elektrische Strom zum Unterdrücken des mikrobiologischen Wachstums innerhalb des Gehäuses (20) konfiguriert ist,
**dadurch gekennzeichnet, dass** das Gehäuse (20) ein rotierendes Gehäuse (20) ist, das so angeordnet ist, dass es um eine Gehäuseachse (56) rotiert, und das System (10) ferner umfasst:
- ein rotierendes Element (68), wobei
das rotierende Element (68) zumindest teilweise innerhalb des Gehäuses (20) positioniert ist, wobei das rotierende Element (68) um eine Achse des rotierenden Elements (72) angeordnet ist, und das rotierende Element (68) und das Gehäuse (20) in einem elektrischen Netzwerk (30), das einen Teil des elektrischen Stromkreises (28) bildet, elektrisch gekoppelt sind.

2. System (10) nach Anspruch 1, wobei das System (10) ferner umfasst:
- einen ersten internen Verbinder (40), der das rotierende Element (68) elektrisch mit dem Gehäuse (20) koppelt, und wobei der erste interne Verbinder (40) ein rotierender elektrischer Verbinder ist.

3. System (10) nach Anspruch 1 oder 2, wobei das rotierende Element (68) umfasst:
- eine Welle des rotierenden Elements (70) und
- einen Rotor (94), wobei
die Welle des rotierenden Elements (70) sich von innerhalb des Gehäuses (20) nach außerhalb des Gehäuses (20) erstreckt und so angeordnet ist, dass sie um die Achse des rotierenden Elements (72) relativ zum Gehäuse (20) rotiert, und der Rotor (94) an der rotierenden Elementwelle (70) befestigt ist und zum Erzeugen einer Strömung des Fluids entlang der Achse des rotierenden Elements (72) bei einer Drehung um die Achse des rotierenden Elements (72) konfiguriert ist.

4. System (10) nach Anspruch 3, wobei das System (10) ferner umfasst:
- einen zweiten internen Verbinder (42), der das rotierende Element (68) elektrisch mit dem Gehäuse (20) koppelt, wobei der zweite interne Verbinder (42) ein rotierender elektrischer Verbinder ist und der Rotor (94) zwischen dem ersten internen Verbinder (40) und dem zweiten internen Verbinder (42) positioniert ist.

5. System (10) gemäß einem der Ansprüche 1 bis 4, wobei das System (10) ferner umfasst:
- ein statisches Element (76), wobei
das statische Element (76) und das Gehäuse (20) in dem elektrischen Netzwerk (30) elektrisch gekoppelt sind.

6. System (10) nach Anspruch 5, wobei das System (10) ferner umfasst:
- einen dritten internen Verbinder (192), der das statische Element (76) elektrisch mit dem rotierenden Element (68) koppelt.

7. System (10) gemäß einem der Ansprüche 1 bis 6, wobei der elektrische Stromkreis (28) mit Masse verbunden ist.

8. System (10) gemäß einem der Ansprüche 1 bis 7, wobei der Strom ein Wechselstrom ist, der Strom eine Rechteckwellenform aufweist, der Strom unter 10 mA liegt und der Strom größer als 0,01 mA ist.

9. System (10) gemäß einem der Ansprüche 1 bis 8, wobei das Gehäuse (20) und das rotierende Element (68) Teil eines Dekanters (214) bilden und der Dekanter (214) so angeordnet ist, dass er bei einer Drehung des Gehäuses (20) eine leichtere Phase des Fluids und eine schwerere Phase des Fluids innerhalb des Gehäuses (20) bildet.

## Revendications

1. Système (10) comprenant :
- un boîtier (20), et
- une source d'alimentation électrique (22), dans lequel
le boîtier (20) est électriquement conducteur et configuré pour contenir un fluide, la source d'alimentation (22) est agencée pour fournir un courant électrique, le boîtier (20) et la source d'alimentation électrique (22) sont couplés de manière opérationnelle dans un circuit électrique (28) agencé pour conduire le courant de la source d'alimentation électrique (22) à travers le boîtier (20), et le courant électrique est configuré pour inhiber la croissance microbiologique à l'intérieur du boîtier (20),
**caractérisé en ce que** le boîtier (20) est un boîtier rotatif (20) agencé pour tourner autour d'un axe de boîtier (56), et le système (10) comprend également :
- un élément rotatif (68), dans lequel
l'élément rotatif (68) est situé au moins en partie à l'intérieur du boîtier (20), l'élément rotatif (68) est agencé pour tourner autour d'un axe d'élément rotatif (72), et l'élément rotatif (68) et le boîtier (20) sont couplés électriquement dans un réseau électrique (30) qui fait partie du circuit électrique (28).

2. Système (10) selon la revendication 1, dans lequel le système (10) comprend également :
- un premier connecteur interne (40) qui couple électriquement l'élément rotatif (68) au boîtier (20), et le premier connecteur interne (40) est un connecteur électrique rotatif.

3. Système (10) selon la revendication 1 ou 2, dans lequel l'élément rotatif (68) comprend :
- un arbre d'élément rotatif (70), et
- un rotor (94), dans lequel
l'arbre d'élément rotatif (70) se prolonge de l'intérieur du boîtier (20) vers l'extérieur du boîtier (20) et est agencé pour tourner autour de l'axe d'élément rotatif (72) par rapport au boîtier (20), et le rotor (94) est fixé à l'arbre d'élément rotatif (70) et configuré pour générer un écoulement du fluide le long de l'axe d'élément rotatif (72) lors d'une rotation autour de l'axe d'élément rotatif (72).

4. Système (10) selon la revendication 3, dans lequel le système (10) comprend également :
- un deuxième connecteur interne (42) qui couple électriquement l'élément rotatif (68) au boîtier (20), le deuxième connecteur interne (42) est un connecteur électrique rotatif, et le rotor (94) est situé entre le premier connecteur interne (40) et le deuxième connecteur interne (42).

5. Système (10) selon l'une quelconque des revendications 1 à 4, dans lequel le système (10) comprend également :
- un élément statique (76), dans lequel
l'élément statique (76) et le boîtier (20) sont couplés électriquement dans le réseau électrique (30).

6. Système (10) selon la revendication 5, dans lequel le système (10) comprend également :
- un troisième connecteur interne (192) qui couple électriquement l'élément statique (76) à l'élément rotatif (68).

7. Système (10) selon l'une quelconque des revendications 1 à 6, dans lequel le circuit électrique (28) est connecté à la masse.

8. Système (10) selon l'une quelconque des revendications 1 à 7, dans lequel le courant est un courant alternatif, le courant a une forme d'onde carrée, le courant est inférieur à 10 mA et le courant est supérieur à 0,01 mA.

9. Système (10) selon l'une quelconque des revendications 1 à 8, dans lequel le boîtier (20) et l'élément rotatif (68) font partie d'un décanteur (214), et le décanteur (214) est agencé pour former une phase plus légère du fluide et une phase plus lourde du fluide à l'intérieur du boîtier (20) lors d'une rotation du boîtier (20).
